Europäisches Patentamt

European Patent Office

Office européen des brevets

(19)

(11) Numéro de publication : **0 234 422 B1**

(12) # FASCICULE DE BREVET EUROPEEN

(45) Date de publication du fascicule du brevet :
09.01.91 Bulletin 91/02

(51) Int. Cl.⁵ : **A61C 19/04, A61C 13/08**

(21) Numéro de dépôt : **87101950.1**

(22) Date de dépôt : **12.02.87**

(54) Procédé d'amélioration de l'albédo d'éléments vivants pour leur analyse optique tridimensionelle.

(30) Priorité : 20.02.86 FR 8603040

(43) Date de publication de la demande :
02.09.87 Bulletin 87/36

(45) Mention de la délivrance du brevet :
09.01.91 Bulletin 91/02

(84) Etats contractants désignés :
AT BE CH DE ES GB GR IT LI LU NL SE

(56) Documents cités :
EP-A- 0 054 785
GB-A- 2 051 396
US-A- 2 115 034
US-A- 4 512 743

(72) Inventeur : **Mezi, Michel**
**62, rue Chabot Charny**
**F-21000 Dijon (FR)**
Inventeur : **Duret, Bernard**
**Les Travers Saint Gervais/Isère**
**F-38470 Vinay (FR)**
Inventeur : **Duret, François**
**Drayes des Vignes**
**F-38690 Le grand lemps (FR)**

(74) Mandataire : **Jörchel, Dietrich R.A. et al**
**c/o BUGNION S.A. Conseils en Propriété**
**Industrielle 10, route de Florissant Case**
**postale 375**
**CH-1211 Genève 12 Champel (CH)**

(73) Titulaire : **LABORATOIRE S.P.A.D.**
**F-21800 Quetigny (FR)**

EP 0 234 422 B1

Jouve, 18, rue Saint-Denis, 75001 PARIS

## Description

Procédé d'amélioration de l'albédo d'éléments vivants pour leur analyse optique tridimensionnelle.

L'invention concerne l'analyse optique tridimensionnel le d'éléments vivants permettant de recueillir les informations nécessaires à la technique de conception et de fabrication assistée par ordinateur, dans le cas, par exemple, de prothèses médicales.

Le document TD 73 publié le 15 novembre 1985, décrit une nouvelle technique pour la réalisation de prothèses fixes -telles que couronnes, bridges ... pour l'art dentaire- qui est susceptible d'être étendue ultérieurement aux prothèses mobiles. Une technique analogue est décrite dans la demande de brevet E-PA-54 785 (HÖRMANN).

Cette technique abandonne la classique prise d'empreinte à l'aide d'alginate ou d'élastomère de silicone : elle est basée sur l'analyse optique tridimensionnelle en lumière cohérente d'une dent devant être reconstituée, ainsi qu'éventuellement de la partie adjacente de la gencive d'une part, de la face occlusale de la dent lui faisant face, d'autre part.

Les informations recueillies sont traitées par un ordinateur qui pilotera une fraiseuse taillant, avec précision, dans un matériau adapté, la prothèse nécessaire.

La reflexibilité du corps humain étant mauvaise, notamment au niveau des structures translucides comme les dents, le problème consiste à trouver un moyen d'améliorer le facteur de luminance lumineuse de la surface considérée.

Le brevet US 4 512 743 (SANTUCCI) décrit un procédé pour appliquer un revêtement sur la surface d'une dent pour masquer les décolorations ou d'autres imperfections similaires. Obtenu par polymérisation in situ d'un monomère acrylique, il comprend une charge et des pigments lui donnant une coloration naturelle. Après durcissement, sa surface est lisse et ne nécessite pratiquement pas de polissage. Son adhésion à la dent est excellente et sa durée de vie dépasse 2 ans.

La présente invention a pour but d'augmenter le rapport entre l'énergie lumineuse réfléchie par l'objet analysé et celle qu'il reçoit, cet objet pouvant être une partie d'un être vivant. Elle permet également une meilleure répartition spatiale de cette énergie lumineuse, tendant à transformer cet objet en un corps lambertien, et évite les phénomènes lumineux parasites dus à des réfractions et réflexions dans la masse de l'objet.

Ce procédé est caractérisé par le fait que l'on recouvre ces parties d'une suspension fluide d'un pigment blanc, minéral, finement dispersé.

Cette suspension est dénuée de toxicité, elle est utilisable dans le domaine biomédical et permet d'obtenir une image de haute définition car elle s'étale en un revêtement fin et régulier.

Elle rend possible l'analyse optique tridimensionnelle de surfaces présentant un mauvais albédo, en s'affranchissant de lasers de puissance élevée, fort onéreux et de maniement très délicat, voire impossible sur des êtres vivants.

Elle facilite également le traitement informatique d'images tridimensionnelles complexes, en s'affranchissant des variations de teinte et de luminosité des surfaces et en atténuant les effets d'ombres. Au niveau des dents, elle évite notamment les réflexions parasites de lumière qui se produisent dans la profondeur de l'émail.

L'invention est exposée ci-après de façon détaillée avec un exemple, non limitatif, d'une préparation contenant un pigment minéral en suspension fluide.

On met en suspension fluide un pigment blanc de fine granulométrie dans une solution hydro-alcoolique d'un agent filmogène.

Il s'agit d'un pigment atoxique de nature minérale, par exemple :

- Oxyde de titane

- Oxyde de magnésium

- Oxyde de zinc

- Carbonate de magnésium

Les qualités d'oxyde de titane de très fine granulométrie, destinées à la réalisation de produits de maquillage, fournissent des résultats très satisfaisants. La proportion d'agent opacifiant est de l'ordre de 8 à 16 %.

On utilise pour mettre en suspension le pigment blanc, un polymère filmogène soluble en milieu hydroalcoolique. L'agent filmogène est une résine acrylique. L'utilisation de la plupart des autres solvants est exclue en milieu biologique.

De préférence, on utilise le "Carboset 525" (marque déposée) de la firme B.F. GOODRICH à CLEVELAND (OHIO).

Cette résine filmogène assure l'adhérence de la préparation sur la surface à étudier, et, grâce à sa souplesse, y dépose un film uniforme, malgré les irrégularités de forme.

On utilise comme solvant pour cette résine acrylique, qui présente l'avantage d'être soluble à la fois en milieu éthanolique et en solution aqueuse alcaline, un mélange d'alcool éthylique et d'eau ammoniaquée. Grâce à la volatilité élevée de l'alcool, la préparation sèche assez rapidement et l'analyse optique peut être réalisée peu de temps après l'application de la suspension fluide ainsi composée.

Toutefois, on limite le titre alcoolique du solvant afin d'éviter l'action irritante sur les tissus vivants d'une solution trop concentrée en éthanol.

On neutralise la résine acrylique par une base volatile comme l'ammoniaque afin d'obtenir un film hydrorésistant.

La proportion d'agent filmogène qu'on utilise est de l'ordre de 8 à 16 %.

On ajoute un mouillant pour faciliter la suspension du pigment et l'étalement de la préparation ainsi constituée en une couche mince et régulière. On utilise de préférence le polysorbate 20 comme agent mouillant.

On atténue la saveur peu agréable de cette préparation en effectuant une aromatisation appropriée : des notes telles que "cerise, banane, tous fruits" sont bien adaptées.

A titre d'exemple, non limitatif, on peut citer une composition centésimale ainsi constituée.

| | |
|---|---|
| Oxyde de titane | 12 |
| Carboset 525 | 12,5 |
| Alcool rectifié à 95° | 45 |
| polysorbate 20 | 0,4 |
| solution d'ammoniaque à 30 % | 1,25 |
| arome Q.S | |
| eau purifiée qsp | 100 gr. |

On opère comme suit pour mélanger les constituants de cette préparation :
– On mélange la solution d'ammoniaque et le polysorbate dans l'eau purifiée,
– On dissout séparément l'arome dans l'alcool,
– On réunit les deux solutions ainsi obtenues et on dissout la résine sous agitation rapide,
– on disperse l'oxyde de titane dans l'ensemble.

Le procédé selon l'invention reçoit différentes applications parmi lesquelles on peut citer sans que cette énumération soit exhaustive :
– une lecture optique tridimensionnelle endobuccale à l'aide de lumière cohérente pour la réalisation de prothèses dentaires selon la technique dite "Conception Fabrication Assistée par Ordinateur" (C.F.A.O) ;
– une étude optique tridimensionnelle de la déformation de certaines structures biologiques, par exemple au niveau du palais pour les prothèses mobiles ;
– une étude optique tridimensionnelle de parties du corps sur lesquelles une prothèse ou un dispositif doit être ajusté avec précision, en chirurgie osseuse notamment.

## Revendications

1. "Procédé pour améliorer l'analyse optique tridimensionnelle de parties d'êtres vivants, caractérisé en ce qu'on améliore l'albédo, et en particulier la répartition spatiale de la lumière renvoyée par réflexion diffuse, desdites parties, en couvrant ces parties d'un film uniforme d'une suspension comprenant un pigment blanc minéral finement dispersé et au moins un agent filmogène".

2. Procédé selon la revendication 1, caractérisé en ce que l'on utilise l'oxyde de titane comme pigment blanc.

3. Procédé selon l'une des revendications 1 ou 2, caractérisé par le fait que l'on utilise une suspension fluide.

4. Procédé selon l'une des revendications 1 à 3, caractérisé en ce que l'on utilise une suspension de pigment contenant un polymère filmogène soluble en milieu hydroalcoolique.

5. Procédé selon la revendication 4, caractérisé en ce que l'on utilise une résine acrylique comme polymère filmogène

6. Procédé selon la revendication 5, caractérisé en ce que l'on utilise une résine acrylique neutralisée par une base volatile, telle l'ammoniaque.

7. Procédé selon l'une des revendications précédentes, caractérisé en ce que l'on utilise une suspension du pigment contenant un agent mouillant facilitant son application sous forme d'une couche mince et régulière.

8. Procédé selon la revendication 7, caractérisé en ce que l'on utilise comme agent mouillant le polysorbate 20.

9. Procédé selon l'une des revendications précédentes, caractérisé en ce l'on utilise une suspension de pigments contenant un arôme.

10. Composition destinée à améliorer l'analyse optique tridimentionnelle de parties d'êtres vivants, selon la revendication 1, caractérisée en ce qu'elle renferme en suspension un pigment blanc minéral finement dispersé et un agent filmogène pour améliorer l'albédo.

## Ansprüche

1. Verfahren zum Verbessern der optischen dreidimensionalen Analyse von Teilen von Lebewesen, dadurch gekennzeichnet, dass man die Albedo dieser Teile und insbesondere die räumliche Verteilung des von diesen Teilen durch diffuse Reflexion zurückgeworfenen Lichts verbessert, indem diese Teile mit einem gleichförmigen Film einer Suspension bedeckt werden, welche ein mineralisches weisses, feinverteiltes Pigment und wenigstens ein filmbildendes Mittel enthält.

2. Verfahren nach Anspruch 1, dadurch gekennzeichnet, dass man Titanoxid als weisses Pigment verwendet.

3. Verfahren nach einem der Ansprüche 1 oder 2, dadurch gekennzeichnet, dass man eine dünnflüssige Suspension verwendet.

4. Verfahren nach einem der Ansprüche 1 bis 3, dadurch gekennzeichnet, dass man eine Pigment-Suspension verwendet, welche ein in einem hydroalkoholischen Mittel lösbares filmbildendes Polymer enthält.

5. Verfahren nach Anspruch 4, dadurch gekennzeichnet, dass man ein Acrylharz als filmbildendes Polymer verwendet.

6. Verfahren nach Anspruch 5, dadurch gekennzeichnet, dass man ein durch eine flüchtige Base wie Ammoniak neutralisiertes Acrylharz verwendet.

7. Verfahren nach einem der vorangehenden Ansprüche, dadurch gekennzeichnet, dass man eine Pigmentsuspension verwendet, die ein Netzmittel enthält, welches ihre Anwendung in Form einer dünnen und regelmässigen Schicht erleichtert.

8. Verfahren nach Anspruch 7, dadurch gekennzeichnet, dass man als Netzmittel Polysorbat 20 verwendet.

9. Verfahren nach einem der vorangehenden Ansprüche, dadurch gekennzeichnet, dass man eine Pigmentsuspension verwendet, die ein Aroma enthält.

10. Zur Verbesserung der optischen dreidimensionalen Analyse von Teilen von Lebewesen gemäss Anspruch 1 bestimmte Verbindung, dadurch gekennzeichnet, dass sie als Suspension ein mineralisches weisses, feinverteiltes Pigment und ein filmbildendes Mittel zur Verbesserung der Albedo enthält.

## Claims

1. Method of improving the three-dimensional optical analysis of portions of living beings, characterized in that the albedo, and in particular the space distribution of the light reflected by diffuse reflection by said portions is improved, wherein said portions are covered with a uniform film of a suspension comprising a finely dispersed mineral white pigment and at least a film forming agent.

2. Method according to claim 1, characterized in that titanium oxide is used as the white pigment.

3. Method according to claim 1 or 2, characterized in that a fluid suspension is used.

4. Method according to one of claims 1, 2, 3 characterized in that a pigment suspension containing a film forming polymer soluble in a water-alcohol solution is used.

5. Method according to claim 4, characterized in that an acrylic resin is used as a film forming polymer.

6. Method according to claim 5, characterized in that an acrylic resin neutralized by a volatile base such as ammonia is used.

7. Method according to anyone of the preceding claims, characterized in that a suspension of the pigment containing a wetting agent facilitating its application in the form of a thin and regular layer is used.

8. Method according to claim 7, characterized in that polysorbate 20 is used as a wetting agent.

9. Method according to anyone of the preceding claims, characterized in that a suspension of pigments containing an aroma is used.

10. A composition for improving the three-dimensional optical analysis of portions of living beings according to claim 1, characterized in that it contains a white mineral pigment finely dispersed in a suspension, and a film forming agent, for improving the albedo.